# FASCICULE DE BREVET EUROPEEN

(11) **EP 2 094 205 B1**
(45) Date de publication et mention de la délivrance du brevet: **15.12.2010**
(21) Numéro de dépôt: 07872405.1
(22) Date de dépôt: 19.12.2007
(51) Int. Cl.: A61F 5/02

(54) **DISPOSITIF DE SOUTIEN EN LORDOSE DU DOS, POUR CORRECTION DE LA POSTURE**
VORRICHTUNG FÜR LORDOSESTÜTZE DES RÜCKENS ZUR HALTUNGSKORREKTUR
DEVICE FOR LORDOSIS SUPPORT OF THE BACK FOR CORRECTING THE POSTURE

(30) Priorité: 19.12.2006 FR 0611073
(43) Date de publication de la demande: 02.09.2009
(73) Titulaire: Faouën, Patrick, 69300 Caluire et Cuire (FR); Salmochi, Jean-François, 69390 Voulres (FR)
(72) Inventeur: Faouën, Patrick, 69300 Caluire et Cuire (FR); Salmochi, Jean-François, 69390 Voulres (FR)
(74) Mandataire: Maureau, Philippe
(86) Numéro de dépôt international: PCT/FR2007/002118
(87) Numéro de publication internationale: WO 2008/093023

(56) Documents cités:
- WO-A-2006/068459
- US-A- 2 808 050
- US-A- 5 127 897
- US-A1- 2005 043 660
- US-A1- 2005 203 453

## Description

La présente invention concerne un dispositif de soutien en lordose du dos, agissant au niveau de la région lombaire du porteur ou de l'utilisateur du dispositif, et permettant ainsi une correction de la posture. Ce dispositif offre des zones d'appui actif au niveau de la région lombaire, de manière à imposer et conserver une position corrigée en lordose, et à limiter les mouvements nocifs de flexion du tronc vers l'avant.

Le document US 2005/203453 A représente l'état de la technique le plus proche et détermine le préambule de la revendication 1.

Le dispositif de soutien en lordose, objet de l'invention, peut notamment prendre la forme d'une ceinture lombaire ou d'un corset orthopédique, entourant le tronc ou la taille de son utilisateur. Il constitue, dans ce cas un dispositif orthopédique. Toutefois, le dispositif de soutien en lordose, objet de l'invention, peut aussi être associé à un siège, plus particulièrement à un dossier de siège par exemple de véhicule automobile, sur lequel s'assoit l'utilisateur, auquel cas le dispositif n'est pas attaché au tronc ou à la taille de l'utilisateur. Dans la suite, l'invention sera exposée en référence à un dispositif orthopédique, sans qu'il en résulte une limitation de la portée de l'invention quant à la structure et aux utilisations du dispositif.

La correction de posture, réalisée par un dispositif du genre ici considéré, consiste en un maintien en arrière du tronc (en rétroposition) avec antéversion pelvienne, c'est-à-dire un basculement du bassin vers l'avant, ce qui implique une mise en lordose au niveau lombaire, autrement dit une augmentation de la courbure lombaire.

D'une manière générale, ou connaît les ceintures et corsets de l'état de la technique, qui ont surtout une action, jusqu'à présent considérée comme bénéfique, de diminution de la lordose, c'est-à-dire de réduction de la courbure à convexité antérieure de la colonne vertébrale lombaire. Les ceintures de maintien lombaire avec des points d'appui distants au niveau dorsal et sacré par exemple ont un effet de diminution de la courbure lombaire.

De telles ceintures de maintien lombaire pouvaient convenir dans le passé car elles allaient dans le sens des anciennes conceptions estimant nocive la lordose lombaire, et visant à favoriser un mouvement de rétroversion du bassin.

Toutefois, ces dispositifs de l'état de la technique ne peuvent s'utiliser en toutes circonstances, car soit les forces de maintien qu'ils sont censés exercer diminuent lors de mouvements brusques ou de forte intensité des personnes portant ces dispositifs, soit ils entraînent des contraintes inconfortables, notamment en position assise.

Pour éviter ces inconvénients, il a été aussi déjà proposé des dispositifs de contention qui, à l'inverse des précédents, visent à favoriser la mise en lordose.

Ainsi, on connaît des dispositifs « lordosants » anciens, qui sont rigides et sont prescrits par les médecins pour la seule indication de traitement de fractures des corps vertébraux dorsaux ou dorso-lombaires. Des exemples de ces dispositifs sont le corset plâtré BOHLER ou les orthèses de type JEWETT. De tels corsets remontent très haut sur l'avant, et ils provoquent ainsi une compression pectorale descendante qui gêne la respiration. Leur force d'appui au niveau lombaire est limitée. L'encombrement de tels dispositifs restreint la pratique de nombreuses activités, notamment sportives.

On connaît aussi des dispositifs « lordosants » plus légers et moins encombrants, qui comportent deux appuis lombaires correctifs symétriques dans un plan frontal, et séparés l'un de l'autre par un espace libe, de manière à venir en appui de part et d'autre de la colonne vertébrale. Les deux appuis ainsi constitués sont montés sur un support commun, maintenu en place par une ou plusieurs sangles entourant le corps du porteur. D'une manière générale, ces deux appuis sont dans certains cas réglables en écartement, dans un plan frontal, mais ils ne possèdent pas de possibilité d'adaptation (dans un plan sagittal c'est-à-dire antéro-postérieur) au profil de la courbure du creux lombaire de l'utilisateur.

Ainsi, le brevet US 2808050 décrit un dispositf pourvu de deux lames d'appui identiques, s'étendant verticalement, qui sont fixées sur un support commun en forme de barre transversale, sans possibilité de réglage en écartement, et sans possibilité de réglage de leur courbure (dans un plan sagittal).

La demande de brevet US 2005/0043660 révèle une ceinture lombaire pourvue, dans sa partie lombaire, de deux poches gonflables d'appui qui prennent place de part et d'autre de la colonne vertébrale. Ce dispositif ne comporte ni réglage en écartement des deux poches, ni possibilité d'adaptation individuelle du rayon de courbure de chaque poche par un réglage différencié de la pression de gonflage des deux poches.

Dans le brevet US 5127897, une plaque support reçoit deux éléments d'appui hémisphériques, disposés de part et d'autre d'un espace libre correspondant à la position de la colonne vertébrale en hauteur, et l'écartement des deux éléments d'appui est réglable sur cette plaque grâce à des lumilères horizontales. Toutefois, le rayon de courbure de ces deux éléments d'appui hémisphériques est invariable par construction même.

Le document WO 2006/068459 décrit un corset orthopédique, pourvu dans sa région postérieure de deux éléments symétriques d'appui lombaire, fixés au corset de manière non réglable, et dont le profil vertical ne peut être modifié.

Un dernier exemple d'éléments d'appui lombaires disposés symétriquement, mais non réglables, peut être trouvé dans le brevet US 6190343.

Certes, les sangles ou ceintures de maintien des dispositifs existants permettent une adaptation aux mensurations de leur porteur et un réglage de la force de serrage, mais elles ne constituent, en aucune manière, un moyen de réglage ou d'adaptation de la courbure des éléments d'appui.

Or, comme cela est bien connu, de fortes différences existent entre les personnes, soit dues à des morphotypes différents, soit provoquées par des épisodes douloureux. Ces différences se traduisent non seulement par des variations importantes au niveau de la courbure lombaire, mais aussi par des asymétries (entre côté droit et côté gauche). Ces différences ne sont pas prises en compte par les dispositifs « lordosants » connus, tels que ceux décrits dans les documents précédemment mentionnés.

Face à cet état de la technique, la présente invention a pour but de fournir un dispositif perfectionné de soutien en lordose du dos, prenant en compte de façon effective toutes les différences entre personnes susceptibles d'utiliser ce dispositif, grâce à des appuis rendus réglables d'une façon judicieuse, dans un objectif d'adaptation et de confort, et aussi d'utilisation variées.

A cet effet, l'invention a essentiellement pour objet un dispositif de soutien en lordose du dos, agissant au niveau de la région lombaire du porteur ou de l'utilisateur du dispositif, et permettant ainsi une correction de la posture, le dispositif comprenant, montés sur support commun, deux éléments d'appui lombaire séparés l'un de l'autre par un espace libre et prévus pour venir en appui de part et d'ature de la colonne vertébrale du porteur ou de l'utilisateur, sans contact avec la ligne des épineuses de la colonne vertébrale, les deux éléments d'appui possédant un profil général arrondi en particulier dans un plan sagittal, ce dispositif étant caractérisé par le fait que les deux éléments d'ppui sont rendus réglables, indépendamment l'un de l'autre, en ce qui concerne leur profil ou leur rayon de courbure.

Ainsi, l'invention apporte aux dispositifs de soutien en lordose du dos, du genre ici concerné, une adaptabilité et une efficacité améliorées, quelle que soit la posture adoptée : penché, accroupi, adossé, allongé ... Le dispositif proposé est en particulier adaptable à toutes morphologies, variant notamment par la taille (hauteur), le tour de taille ou la longueur de poitrine, ainsi que (du point de vue thérapeutique) à diverses pathologies vertébrales : lombalgie par tassement de disques intervertébraux, asymétrie posturale (scoliose), spondyloslistésis. Le dispositif, convenablement ajusté, stabilise le tronc tout à la fois dans le plan sagittal et dans le plan frontal. Il limite les contraintes physiologiques rencontrées avec les corsets lordosants classique, en évitant toute gêne respiratoire et en ne limitant pas l'extension et la rotation du tronc. Le réglage des éléments d'appui, associé à une réalisation de ces éléments dans des matériaux appropriés, contribue aussi au confort du porteur ou de l'utilisateur du dispositif. Enfin, ce dispositif permet de limiter des efforts physiques, lors de l'exercice d'activités physiques ou sportives.

Dans une forme de réalisation avantageuse du disposiitf de soutien en lordose du dos, objet de l'invention, les deux éléments d'appui lombaire sont montés sur un support commun en forme de cadre ou de plaque ajourée. L'ouverture du cadre, ou les ajours de la plaque, donnent un accès aisé aux deux éléments d'appui lombaire, pour réaliser les réglages de position et/ou de rayon de courbure de ces éléments d'appui, sans que l'utilisateur ait besoin de retirer le dispositif de son dos. Une telle réalisation ajourée a aussi l'avantage d'être légère.

Selon un mode de réalisation de l'invention, les deux éléments d'appui lombaire comprennent respectivement deux lames ressort, de profil incurvé, montées séparément sur le support commun notamment en forme de cadre ou de plaque ajourée, les deux lames ressort étant de préférence rendues réglables, en ce qui concerne leur profil ou leur rayon de courbure, l'une indépendamment de l'autre.

Les deux lames ressort sont prévues pour venir en appui dans la région lombaire du porteur ou de l'utilisateur du dispositf, de part et d'autre de la colonne vertébrale. Le profil ou le rayon de courbure de chaque lame ressort peut être modifié, indépendamment du profil ou du rayon de courbure de l'autre lame :
- soit par modification de la position du point d'accrochage de l'une au moins des extrémités de la lame ressort sur le support tel que cadre ou plaque, en particulier en prévoyant sur ledit support des crans ou des rainures crantées pour l'accrochage, dans une position choisie, de l'extrémité mobile de chaque lame ressort ;
- soit par une vis de pression, orientée perpendiculairement à la lame ressort et montée sur le support tel que cadre ou plaque, la vis de pression pouvant ainsi être réglée axialement et/ou dans le sens de la hauteur.

Ces dispositions permettent de modifier la courbure de chacune des lames ressort, dans le plan de leur profil (plan sagittal).

La réalisation des éléments d'appui sous la forme de deux lames ressort permet aussi de modifier aisément la distance horizontale entre les deux lames ressort, en prévoyant des moyens de réglage de la position de serrage d'une extrémité de chaque lame ressort sur le support tel que cadre ou plaque, tandis que l'autre extrémité de cette lame prend une position correspondante, notamment par coulissement dans une rainure crantée du support. L'écartement des deux éléments d'appui peut ainsi être ajusté, par l'utilisateur du dispositif lui-même, en fonction de sa morphologie, de sa pathologie ou encore de sa sensation de confort.

Dans la forme de réalisation la plus simple, les deux lames ressort constituent, à elles seules, les deux éléments d'appui lombaire, venant dans ce cas directement en contact avec le dos du porteur ou de l'utilisateur.

Selon une autre possibilité, au moins un élément d'appui complémentaire, en matière souple ou élastique, est monté sur chaque lame ressort, de préférence dans une position modifiable le long de cette lame ressort, auquel cas c'est le ou les éléments d'appui complémentaires qui viennent en contact avec le dos du porteur ou de l'utilisateur.

Un ou deux éléments d'appui complémentaires, réalisés en matière souple ou élastique, par exemple du type poche gonflable ou corps visco-élastique, sont ainsi fixés sur chaque lame ressort, ce qui assure notamment une répartition confortable de la force d'appui sur la surface du dos. Ces éléments d'appui complémentaires peuvent être réglés en position de long des lames ressort, donc en hauteur, par différents moyens tels que :
- fixation modifiable en position par un système autoagrippant, ou
- encliquetage sur des crans prévus sur la longueur de chaque lame ressort, ou
- coulissement le long de chaque lame, par exemple au moyen d'une bague.

La présence de tels éléments d'appui complémentaires permet une meilleure adaptation au profil de la colonne vertébrale, notamment dans le plan sagittal, au niveau lombaire. Le positionnement en hauteur de ces éléments d'appui complémentaires permet à la fois de modifier le profil ou le rayon de courbure global des parties d'appui du dispositif, tel qu'il résulte des lames et des éléments d'appui complémentaires, et de modifier les points d'application des forces de pression exercées sur le dos, tout en optimisant la sensation de confort.

Les éléments d'appui complémentaires peuvent être prévus escamotables, notamment par rabattement contre la face arrière (éloignée du dos de l'utilisateur) de chaque lame ressort, selon l'évolution du traitement ou encore selon les positions adoptées par l'utilisateur.

Dans une forme de réalisation particulière, deux éléments d'appui complémentaires sont fixés à des hauteurs différentes sur chaque lame ressort, chaque élément d'appui complémentaire possédant un profil de forme sensiblement triangulaire, et étant de préférence monté dans le sens inverse de l'autre élément d'appui complémentaire. Ainsi, les deux éléments se complètent mutuellement et, selon leur sens de montage, ils contribuent à augmenter au contraire à diminuer le rayon de courbure global de chaque partie d'appui lombaire, tout en modifiant la répartition et l'intensité des forces d'appui exercées sur le dos de l'utilisateur, pour optimiser la sensation de confort.

De plus, les éléments d'appui complémentaires, ou du moins leurs surfaces d'appui sur le dos de l'utilisateur, peuvent être rendus réglables en profondeur :
- soit par gonflage avec de l'air, dans le cas d'éléments d'appui complémentaires du type poche gonflable, au moyen d'une poire de gonflage individuelle pour chaque poche ;
- soit par modification (par les moyens déjà définis plus haut) du profil ou du rayon de courbure de la lame ressort sur laquelle sont montés ces éléments d'appui complémentaires.

Selon la matière constitutive desdits éléments d'appui complémentaires, ceux-ci peuvent aussi jouer utilement un rôle d'amortisseur. Un revêtement ou état de surface particulier des lames ressort, ou des éléments d'appui complémentaires placés sur ces lames, peut en outre procurer un effet bénéfique de massage, par exemple lors du mouvement de marche ou durant la conduite automobile. Cet effet de massage peut être accentué par l'ajout d'un dispositif de mise en vibration.

Dans une variante, l'effet amortisseur peut être obtenu avec des lames ressort dont une extrémité est laissée libre, toutefois de préférence avec un guidage et/ou une butée limitant la déformation.

L'effet amortisseur permet d'adapter le dispositif aux variations de la lordose selon les positions de l'utilisateur, en particulier en appui contre un dossier de siège ou allongé sur un lit.

Comme le conçoit facilement, le dispositif de soutien en lordose du dos, objet de l'invention, peut être incorporé à une ceinture lombaire ou à un corset orthopédique, porté par l'utilisateur. Ce dispositif peut aussi être équipé d'une ou plusieurs sangles spécifiques de maintien, prévues pour entourer le tronc de son porteur, ces sangles pouvant réunir le cadre ou la plaque support du dispositif à au moins un élément antérieur abdominal et/ou costal, du genre plaque, formant contre-appui et pouvant fournir un support au tronc notamment dans une position penchée vers l'avant, cet élément antérieur pouvant par exemple être appuyé contre le bord d'une table.

Dans tous les cas, le dispositif de l'invention, avec ses deux parties d'appui de profil général arrondi, s'appliquant dans la région du creux lombaire de part et d'autre de la colonne verticale, procure les avantages suivants :
- une mise en lordose efficace,
- un effet antalgique de type inhibiteur tactile,
- un rappel sensoriel permettant de conserver une posture corrigée,
- une absence de contact avec les épineuses.

A cet égard, on notera que la présente invention apporte les appuis nécessaires à la prise de conscience par l'utilisateur des principes d'économie gestuelle, en particulier de la bonne utilisation de la mobilité de l'articulation des hanches. En effet, la qualité des informations sensorielles, tactiles et proprioceptives est un aspect essentiel des aides orthopédiques, souvent même décrit comme fondamental pour obtenir un effet préventif autant que correctif.

L'invention sera de toute façon mieux comprise à l'aide de la description qui suit, en référence au dessin schématique annexé représentant, à titre d'exemples, quelques formes de réalisation de ce dispositif de soutien en lordose du dos :
Figure 1 est une vue de face d'un dispositif de soutien en lordose du dos, conforme à la présente invention :
Figure 2 est une vue par l'arrière du dispositif de la figure 1 ;
Figure 3 est une vue en coupe verticale du dispositif des figures 1 et 2, dans une première configuration ;
Figure 4 est une vue en coupe similaire à la figure 3, illustrant une deuxième configuration ;
Figure 5 est une vue en coupe similaire aux précédentes, relative à une variante du dispositif ;
Figure 6 représente, en vue de profil, dans deux positions, la lame seule d'un élément d'appui du dispositif des figures précédentes, avec ses moyens de réglage ;
Figure 7 illustre, en vue de profil, une première variante des moyens de réglage de la lame ;
Figure 8 illustre, en vue de profil une deuxième variante des moyens de réglage de la lame ;
Figure 9 représente, en coupe verticale, une forme de réalisation d'un dispositif utilisant des éléments d'appui gonflables ;
Figure 10 représente, vu schématiquement en coupe horizontale, un dispositif conforme à l'invention utilisant des éléments d'appui tournés à 90° par rapport aux dispositifs des figures précédentes.

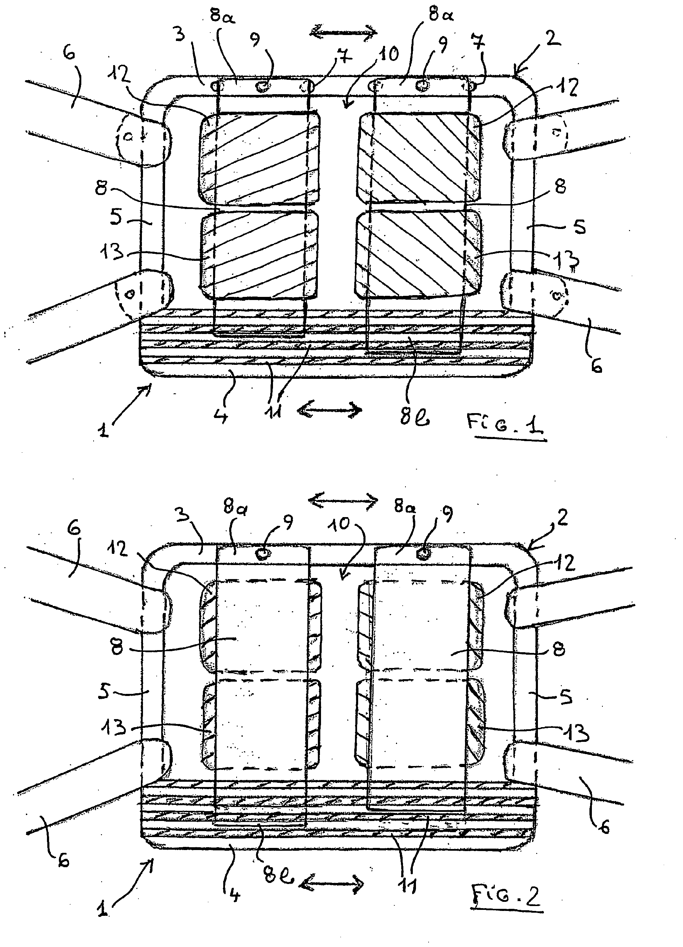

En se référant d'abord aux figures 1 à 6, il est représenté un dispositif de soutien en lordose du dos, désigné dans son ensemble par la référence numérique 1, qui comporte un support en forme de cadre rectangulaire 2, qui en position normale d'utilisation (position considérée dans toute la suite) est situé dans un plan sensiblement vertical. Le cadre 2 comporte un bord supérieur 3 sensiblement horizontal, un bord inférieur 4 sensiblement horizontal, et deux bords 5 verticaux, respectivement droit et gauche. Dans l'exemple illustré (voir figures 1 et 2), le dispositif de soutien en lordose du dos est équipé de plusieurs sangles 6 de maintien, qui sont rattachées aux bords verticaux 5 du cadre 2, et qui sont prévues pour entourer le tronc de la personne portant ce dispositif 1 (personne désigée dans toute la suite de la description comme « porteur »).

Le bord supérieur 3 du cadre 2 comporte deux séries de trous 7 disposées symétriquement, pour la fixation réglable de deux lames ressort 8, de profil incurvé. Les extrémités supérieures 8a respectives des deux lames ressort 8 sont fixées dans les trous 7 sélectionnés, au moyen de vis 9. Un espace libre central 10 sépare les deux lames ressort 8.

Le bord inférieur 4 du cadre 2 comporte plusieurs rainures crantées 11 parallèles et horizontales. L'une de ces rainures 11 est sélectionnée pour recevoir et retenir l'extrémité inférieure 8b de chaque lame ressort 8.

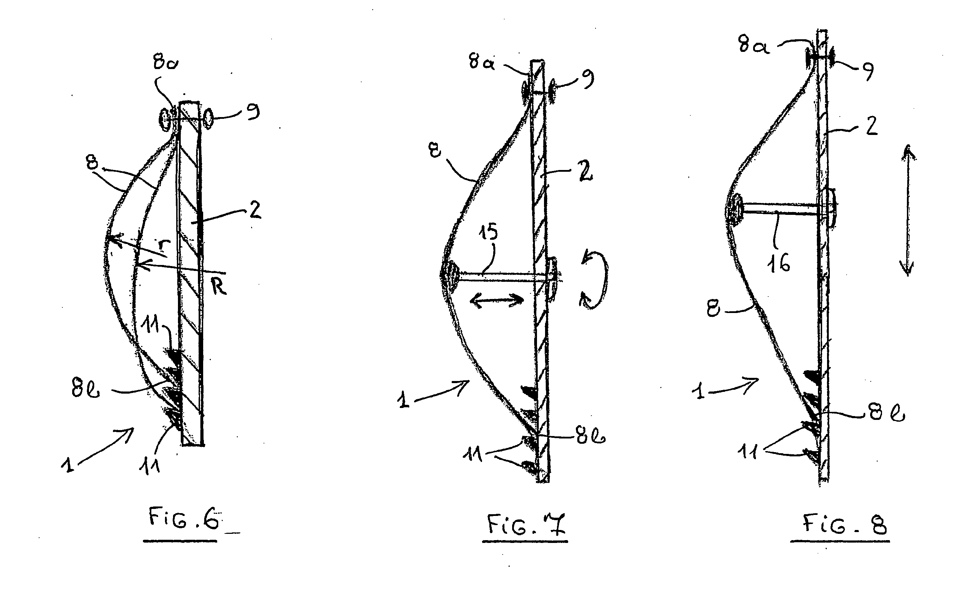

Selon la rainure 11 sélectionnée, et comme l'illustre la figure 6, le profil de la lame ressort 8 et son rayon de courbure sont modifiés. Ainsi, le rayon de courbure peut posséder une valeur élevée R ou une valeur réduite r. De plus, la position de l'apex de la lame ressort 8 est modifiée, c'est-à-dire déplacée vers le haut ou vers le bas. Comme on le comprend aisément, le réglage du profil et du rayon de courbure s'effectue de façon indépendante, pour chaque lame ressort 8, puisque l'extrémité inférieure 8b d'une lame 8 peut être accrochée dans une rainure différente de la rainure recevant l'extrémité inférieure 8b de l'autre lame 8.

Dans la configuration la plus simple, les deux lames ressort 8 disposées et se déformant chacune dans un plan « sagittal » (autrement dit un plan antéro-postérieur, par référence au porteur), constituent respectivement deux éléments d'appui lombaire, réglables indépendamment l'un de l'autre, qui viennent directiment en contact avec le dos du porteur, dans la région du creux lombaire. L'espace libre 10 ménagé entre les deux lames ressort 8 évite tout contact du dispositif 1 avec la ligne des épineuses de la colonne vertébrale du porteur.

Par le choix du trou 7 dans lequel est fixée l'extrémité supérieure 8a de chaque lame ressort 8, on règle aussi l'écartement des deux lames 8, de façon indépendante pour chaque lame, l'extrémité inférieure 8b de cette lame prenant une position correspondante par coulissement dans l'une des rainures crantées 11 du cadre 2.

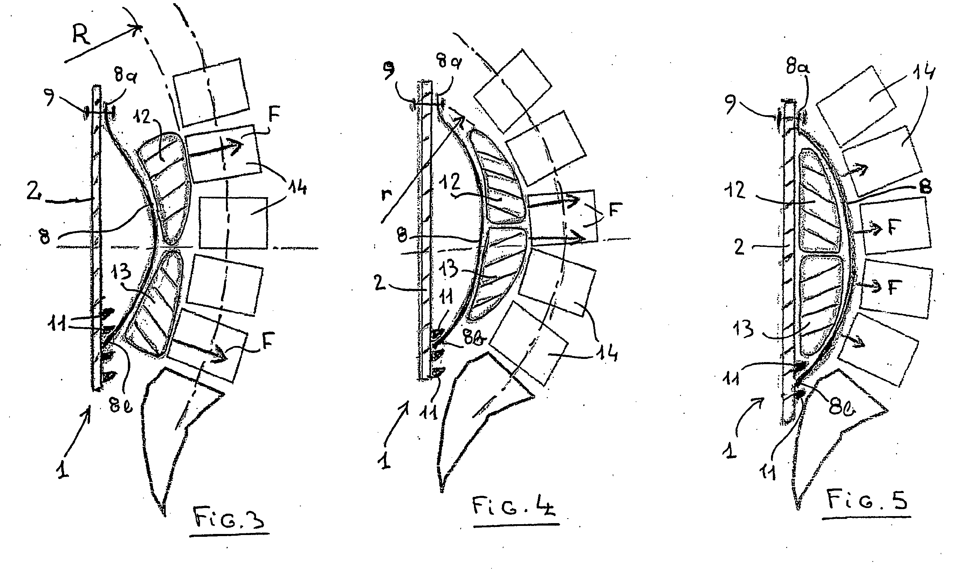

Toutefois, dans un mode de réalisation plus sophistiqué chaque lame ressort 8 porte deux éléments d'appui complémentaire 12 et 13, en matière souple, qui sont prévus pour venir en contact avec le dos du porteur. Comme le montrent notamment les figures 3 et 4, les deux éléments d'appui complémentaires 12 et 13 possèdent (dans un plan vertical) un profil de forme générale triangulaire, et il sont superposés avec un montage en sens inverse l'un de l'autre. Plus particulèrement, dans la configuration selon la figure 3, les deux éléments d'appui complémentaires 12 et 13 portés par la lame 8 sont rapprochés par leur « pointe » si bien que le rayon de courbure global R est augmenté. Au contraire, dans la configuration selon la figure 4, les deux éléments d'appui complémentaires 12 et 13 portés par la lame 8 sont rapprochés par leur « base » si bien que le rayon de courbure global r est diminué. Le coulissement des éléments d'appui complémentaires 12 et 13 le long des lames ressort 8 procure un degré de réglage supplémentaire, dans le sens de la hauteur.

Dans une variante, illustrée par la figure 5, les deux éléments d'appui complémentaires 12 et 13 prennent place sous la lame 8, et peuvent ainsi servir d'éléments amortisseurs.

Dans tous les cas, lorsque les sangles 6 de maintien sont serrées autour du tronc du porteur, les éléments d'appui constitués par les lames ressort 8 elles-mêmes et/ou par les éléments d'appui complémentaires 12, 13 exercent, dans le creux des vertèbres lombaires 14, des forces de pression telles qu'indiquées symboliquement par des flèches F.

La figure 7, sur laquelle une lame ressort 8 est représentée sans ses éventuels éléments d'appui complémentaires, indique pour le réglage du profil ou du rayon de courbure de cette lame 8 des moyens constitués par une vis de pression 15, montée au travers du cadre 2 et agissant dans la région médiane de la lame 8. La rotation de la vis 15 s'accompagne ici d'un mouvement axial de celle-ci, repoussant plus ou moins vers l'avant la région médiane de la lame 8 pour augmenter ou diminuer le rayon de courbure de cette lame 8. La vis 15 peut traverser une lumière horizontale du cadre 2 pour permettre aussi le réglage de position transversal de la lame 8.

La figure 8 représente, de manière analogue, une lame ressort 8 pourvue de moyens de réglage autres, constitués par une vis de pression 16 réglable dans le sens de la hauteur, ce qui modifie le profil de la lame 8 et, en particulier, la position de son apex.

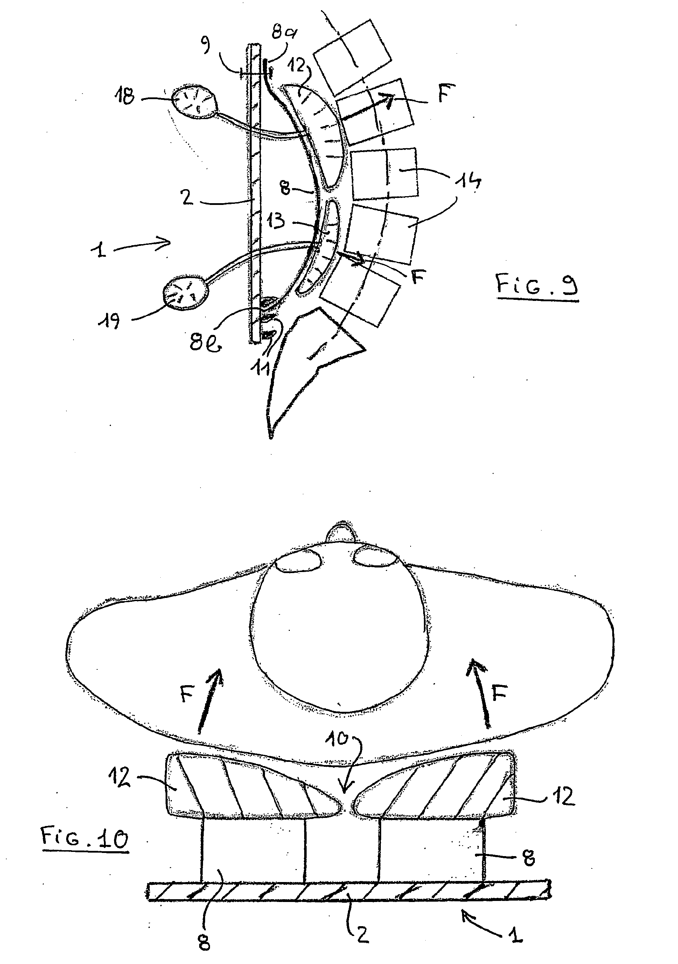

Dans la variante selon la figure 9, les éléments d'appui complémentaires 12 et 13, portés par la lame ressort 8 sont des éléments du type poche gonflable. Chaque poche gonflable comporte une poire de gonflage individuelle, respectivement 18 ou 19. Les moyens de gonflage sont aussi indépendants, du côté droit d'une part et du côté gauche d'autre part.

Enfin, la figure 10 représente une variante dans laquelle des éléments d'appui complémentaires tels que ceux repérés 12, toujours portés par les lames ressort 8 et ici encore de forme sensiblement triangulaire, sont tournés de 90° par rapport aux configurations précédamment décrites. Cette orientation particulière des éléments d'appui complémentaires 12 permet de modifier la répartition et l'intensité des forces d'appui F exercées sur le tronc du porteur, et d'adapter le profil du dispositif 1 dans le sens transversal. On notera que la disposition des éléments d'appui 12 peut être ici symétrique ou dissymétrique.

Comme il va de soi, l'invention ne se limlite pas aux seules formes d'exécution de ce dispositif de soutien en lordose du dos qui ont été décrites ci-dessus, à titre d'exemples ; elle en embrasse, au contraire, toutes les variantes de réalisation et d'application respctant le même principe. C'est ainsi, notamment, que l'on ne s'éloignerait pas du cadre de l'invention, telle que définie dans les revendications annexées :
- en réalisant le support sous toute forme appropriée, telle qu'une plaque ajourée ou non, en lieu et place d'un cadre,
- en réalisant les lames ressort en tous matériaux appropriés, tels que métal, matière plastique ou matériaux composites,
- dans le cas de la présence d'éléments d'appui complémentaires, en réalisant aussi ces éléments en tous matériaux et avec toutes formes appropriées,
- en prévoyant tous moyens pour le réglage du profil ou du rayon de courbure des éléments d'appui, tel que les lames ressort, ou pour le positionnement des éléments d'appui complémentaires,
- dans le cas d'un dispositif équipé de sangles de maintien, en modifiant le nombre et/ou le trajet de ces sangles sur ces lames,
- au lieu de l'utilisation de sangles spécifiques, en incorporant le dispositif à une ceinture lombaire ou à un corset orthopédique,
- en ajoutant au dispositif tous accessoires ou fonctions auxiliaires ; mise en vibration, variateur de pression dans le cas de poches gonflables, motorisations des réglages etc.,
- en augmentant le nombre des supports tels que cadres, et des éléments d'appui tels que lames, pour offrir des appuis additionnels au niveau du tronc, en particulier pour des dispositifs incorporés à des ceintures lombaires ouà des corsets orthopédiques, les cadres supports pouvant être ainsi superposés dans le sens de la hauteur, ou juxtaposés dans le sens de la largeur, les cadres pouvant être liés entre eux au moyen de sangles ou d'articulations de type charnière,
- en concevant le dispositif de soutien en lordose du dos, objet de l'invention, comme un dispositif à associéer à un siège, en particulier à un dossier de siège.

## Revendications

1. Dispositif de soutien en lordose du dos, agissant au niveau de la région lombaire du porteur ou de l'utilisateur du dispositif (1), et permettant ainsi une correction de la posture, le dispositif (1) comprenant, montés sur un support (2) commun, deux éléments d'appui lombaire (8) séparés l'un de l'autre par un espace libre (10) et prévus pour venir en appui de part et d'autre de la colonne vertébrale (14) du porteur ou de l'utilisateur, sans contact avec la ligne des épineuses de la colonne vertébrale, les deux éléments d'appui (8) possédant un profil général arrondi en particulier dans un plan sagittal, **caractérisé en ce que** les deux éléments d'appui (8) sont rendus réglables indépendamment l'un de l'autre, en ce qui concerne leur profil ou leur rayon de courbure (R, r).

2. Dispositif selon la revendication 1, **caractérisé en ce que** les deux éléments d'appui lombaire (8) sont montés sur un support commun (2) en forme de cadre ou de plaque ajourée.

3. Dispositif selon la revendication 1 ou 2, **caractérisé en ce que** les deux éléments d'appui lombaire comprennent respectivement deux lames ressort (8), de profil incurvé, montées séparément sur le support commun (2) notamment en forme de cadre ou de plaque ajourée, les deux lames ressort (8) étant de préférence rendues réglables en ce qui concerne leur profil ou leur rayon de courbure (R, r), l'une indépendamment de l'autre.

4. Dispositif selon la revendication 3, **caractérisé en ce que** le profil ou le rayon de courbure (R, r) de chaque lame ressort (8) est modifiable, indépendamment du profil ou du rayon de courbure de l'autre lame (8), par modification de la position du point d'accrochage de l'une au moins des extrémités (8b) de la lame ressort (8) sur le support (2) tel que cadre ou plaque.

5. Dispositif selon la revendication 4, **caractérisé en ce que** sont prévus, sur ledit support (2) tel que cadre ou plaque, des crans ou des rainures crantées (11) pour l'accrochage, dans une position choisie, de l'extrémité mobile (8b) de chaque lame ressort (8).

6. Dispositif selon la revendication 3, **caractérisé en ce que** le profil ou le rayon de courbure de chaque lame ressort (8) est modifiable, indépendamment du profil ou du rayon de courbure de l'autre lame (8), par une vis de pression (15, 16) orientée perpendiculairement à la lame ressort (8) et montée sur le support (2) tel que cadre ou plaque, la vis de pression (15, 16) pouvant ainsi être réglée axialement et/ou dans le sens de la hauteur.

7. Dispositif selon l'une quelconque des revendications 3 à 6, **caractérisé en ce que** la distance horizontale ente les deux lames ressort (8) est réglable, par des moyens de réglage (7) de la position de serrage d'une extrémité (8a) de chaque lame ressort (8) sur le support (2) tel que cadre ou plaque, tandis que l'autre extrémité (8b) de cette lame (8) prend une position correspondante, notamment par coulissement dans une rainure crantée (11) du support (2).

8. Dispositif selon l'une quelconque des revendications 3 à 7, **caractérisé en ce que** les deux lames ressort (8) constituent, à elles seules, les deux éléments d'appui lombaire, et viennent directement en contact avec le dos du porteur ou de l'utilisateur.

9. Dispositif selon l'une quelconque des revendications 3 à 7, **caractérisé en ce qu'**au moins un élément d'appui complémentaire (12, 13), en matière souple ou élastique, est monté sur chaque lame ressort (8) de préférence dans une position modifiable le long de cette lame ressort (8), le ou les éléments d'appui complémentaires (12, 13) venant en contact avec le dos du porteur ou de l'utilisateur.

10. Dispositif selon la revendication 9, **caractérisé en ce qu'**un ou deux éléments d'appui complémentaires (12, 13), réalisés en matière souple ou élastique, par exemple du type poche gonflable au corps visco-élastique sont fixés sur chaque lame ressort (8).

11. Dispositif selon la revendication 10, **caractérisé en ce que** les éléments d'appui complémentaires (12, 13) sont réglables en position le long des lames ressort (8), donc en hauteur, par une fixation modifiable en positon par un système autoagrippant, ou par un encliquetage sur des crans prévus sur la longueur de chaque lame ressort (8), ou par coulissement le long de chaque lame (8), par exemple au moyen d'une bague.

12. Dispositif selon l'une quelconque des revendications 9 à 11, **caractérisé en ce que** les éléments d'appui complémentaires (12, 13) sont escamotables, notamment par rabattement contre la face arrière de chaque lame ressort (8).

13. Dispositif selon l'une quelconque des revendications 9 à 12, **caractérisé en ce que** les éléments d'appui complémentaires (12, 13) sont fixés à des hauteurs différentes sur chaque lame ressort (8), chaque élément d'appui complémentaire (12, 13) possédant un profil sensiblement triangulaire, et étant de préférence monté dans le sens inverse de l'autre élément.

14. Dispositif selon l'une quelconque des revendications 9 à 13, **caractérisé en ce que**, dans le cas d'éléments d'appui complémentaires (12, 13) du type poche gonflable, ceux-ci sont rendus réglables en profondeur au moyen d'une poire de gonflage individuelle (18, 19) pour chaque poche.

15. Dispositif de soutien en lordose selon l'une quelconque des revendications 1 à 14, **caractérisé en ce qu'**il est incorporé à une ceinture lombaire ou à un corset orthopédique.

16. Dispositif de soutien en lordose selon l'une quelconque des revendications 1 à 14, **caractérisé en ce qu'**il est équipé d'une ou plusieurs sangles spécifiques (6) de maintien, prévues pour entourer le tronc de son porteur.

17. Dispositif de soutien en lordose selon l'une quelconque des revendications 1 à 14, **caractérisé en ce qu'**il est prévu pour être associé à un siège, plus particulier à un dossier de siège par exemple de véhicule automobile.

## Claims

1. Device for lordosis support of the back, acting in the lumbar region of the wearer or user of the device (1) and thus allowing posture correction, the device (1) comprising, mounted on a common support (2), two lumbar bearing elements (8) which are separated from one another by a free space (10) and are intended to come to bear on each side of the vertebral column (14) of the wearer or user, without making contact with the spine of the vertebral column, the two bearing elements (8) having a general profile that is rounded in particular in a sagittal plane, **characterised in that** the two bearing elements (8) can be adjusted independently of one another with regard to their profile or their radius of curvature (R, r).

2. Device according to claim 1, **characterised in that** the two lumbar bearing elements (8) are mounted on a common support (2) in the form of a frame or perforated plate.

3. Device according to claim 1 or 2, **characterised in that** the two lumbar bearing elements comprise respectively two spring leafs (8), of curved profile, which are mounted separately on the common support (2) in particular in the form of a frame or perforated plate, the two spring leafs (8) preferably being adjustable independently of one another with regard to their profile or their radius of curvature (R, r).

4. Device according to claim 3, **characterised in that** the profile or the radius of curvature (R, r) of each spring leaf (8) can be modified, independently of the profile or the radius of curvature of the other leaf (8), by modifying the position of the point of attachment of at least one of the ends (8b) of the spring leaf (8) to the support (2) such as a frame or plate.

5. Device according to claim 4, **characterised in that** notches or notched grooves (11) are provided on said support (2), such as a frame or plate, for attaching in a selected position the movable end (8b) of each spring leaf (8).

6. Device according to claim 3, **characterised in that** the profile or the radius of curvature of each spring leaf (8) can be modified, independently of the profile or the radius of curvature of the other leaf (8), by means of a pressure screw (15, 16) oriented perpendicular to the spring leaf (8) and mounted on the support (2) such as a frame or plate, the pressure screw (15, 16) thus being able to be adjusted axially and/or in the height direction.

7. Device according to any one of claims 3 to 6, **characterised in that** the horizontal distance between the two spring leafs (8) is adjustable by means (7) for adjusting the position at which one end (8a) of each spring leaf (8) is clamped to the support (2) such as a frame or plate, while the other end (8b) of this leaf (8) assumes a corresponding position, in particular by sliding in a notched groove (11) of the support (2).

8. Device according to any one of claims 3 to 7, **characterised in that** the two spring leafs (8) alone constitute the two lumbar bearing elements and come directly into contact with the back of the wearer or user.

9. Device according to any one of claims 3 to 7, **characterised in that** at least one additional bearing element (12, 13), made from flexible or elastic material, is mounted on each spring leaf (8), preferably in a variable position along this spring leaf (8), the additional bearing element(s) (12, 13) coming into contact with the back of the wearer or user.

10. Device according to claim 9, **characterised in that** one or two additional bearing elements (12, 13), made from flexible or elastic material, for example of the inflatable pouch type having a viscoelastic body, are fixed to each spring leaf (8).

11. Device according to claim 10, **characterised in that** the additional bearing elements (12, 13) are adjustable in terms of their position along the spring leafs (8), and therefore in the height direction, by being adjustably fixed in position by a Velcro-type system, or by a snap-fastening into notches provided along the length of each spring leaf (8), or by sliding along each leaf (8), for example by means of a ring.

12. Device according to any one of claims 9 to 11, **characterised in that** the additional bearing elements (12, 13) can be folded away, in particular by being folded back against the rear face of each spring leaf (8).

13. Device according to any one of claims 9 to 12, **characterised in that** the additional bearing elements (12, 13) are fixed at different heights to each spring leaf (8), each additional bearing element (12, 13) having a substantially triangular profile and being preferably mounted in the opposite direction to the other element.

14. Device according to any one of claims 9 to 13, **characterised in that**, in the case of additional bearing elements (12, 13) of the inflatable pouch type, these are adjustable in the depth direction by means of an individual inflation bulb (18, 19) for each pouch.

15. Lordosis support device according to any one of claims 1 to 14, **characterised in that** it is incorporated in a lumbar belt or an orthopaedic corset.

16. Lordosis support device according to any one of claims 1 to 14, **characterised in that** it is equipped with one or more specific retaining straps (6) intended to surround the trunk of the wearer.

17. Lordosis support device according to any one of claims 1 to 14, **characterised in that** it is intended to be associated with a seat, more particularly with a seat back for example of a motor vehicle.

## Patentansprüche

1. Vorrichtung für Lordosestütze des Rückens, die auf Ebene der Lumbalregion des Trägers oder des Benutzers der Vorrichtung (1) agiert und damit eine Korrektur der Haltung erlaubt, wobei die Vorrichtung (1) zwei durch einen Freiraum (10) voneinander getrennte Lumbal-Abstützelemente (8) umfasst, die auf einer gemeinsamen Halterung (2) montiert sind und vorgesehen, um auf der einen und der anderen Seite der Wirbelsäule (14) des Trägers oder des Benutzers ohne Kontakt mit der Linie der Dornfortsätze der Wirbelsäule abzustützen, wobei die zwei Abstützelemente (8) ein allgemein abgerundetes Profil besitzen, insbesondere auf sagittaler Ebene, **dadurch gekennzeichnet, dass** die zwei Abstützelemente (8) voneinander unabhängig einstellbar ausgebildet sind, was ihr Profil oder ihren Krümmungsradius (R, r) betrifft.

2. Vorrichtung nach Anspruch 1, **dadurch gekennzeichnet, dass** die zwei Lumbal-Abstützelemente (8) auf einer gemeinsamen Halterung (2) in Form eines Rahmens oder einer durchbrochenen Platte montiert sind.

3. Vorrichtung nach Anspruch 1 oder 2, **dadurch gekennzeichnet, dass** die zwei Lumbal-Abstützelemente jeweils zwei Federlamellen (8) mit einem gekrümmten Profil umfassen, die separat auf der gemeinsamen Halterung (2) insbesondere in Form eines Rahmens oder einer durchbrochenen Platte montiert sind, wobei die zwei Federlamellen (8) vorzugsweise im Hinblick auf ihr Profil oder ihren Krümmungsradius (R, r) unabhängig voneinander einstellbar ausgebildet sind.

4. Vorrichtung nach Anspruch 3, **dadurch gekennzeichnet, dass** das Profil oder der Krümmungsradius (R, r) jeder Federlamelle (8) unabhängig vom Profil oder vom Krümmungsradius der anderen Lamelle (8) durch Änderung der Position des Festhakpunkts mindestens eines der Enden (8b) der Federlamelle (8) auf der Halterung (2) wie zum Beispiel Rahmen oder Platte veränderbar ist.

5. Vorrichtung nach Anspruch 4, **dadurch gekennzeichnet, dass** auf der Halterung (2) wie zum Beispiel Rahmen oder Platte Kerben oder gekerbte Rillen (11) zum Festhaken des bewegbaren Endes (8b) jeder Federlamelle (8) in einer ausgewählten Position vorgesehen sind.

6. Vorrichtung nach Anspruch 3, **dadurch gekennzeichnet, dass** das Profil oder der Krümmungsradius (R, r) jeder Federlamelle (8) unabhängig vom Profil oder vom Krümmungsradius der anderen Lamelle (8) durch eine senkrecht zur Federlamelle (8) ausgerichtete und auf der Halterung (2) wie zum Beispiel Rahmen oder Platte montierte Druckschraube (15, 16) veränderbar ist, wobei die Druckschraube (15, 16) somit axial und/oder in Richtung der Höhe einstellbar ist.

7. Vorrichtung nach einem der Ansprüche 3 bis 6, **dadurch gekennzeichnet, dass** der horizontale Abstand zwischen den zwei Federlamellen (8) durch Einstellmittel (7) der Feststellposition eines Endes (8a) jeder Federlamelle (8) auf der Halterung (2) wie zum Beispiel Rahmen oder Platte einstellbar ist, wogegen das andere Ende (8b) dieser Lamelle (8) eine entsprechende Position einnimmt, vor allem durch Gleiten in einer gekerbten Rille (11) der Halterung (2).

8. Vorrichtung nach einem der Ansprüche 3 bis 7, **dadurch gekennzeichnet, dass** die zwei Federlamellen (8) für sich allein die zwei Lumbal-Abstützelemente darstellen und direkt mit dem Rücken des Trägers oder des Benutzers in Berührung kommen.

9. Vorrichtung nach einem der Ansprüche 3 bis 7, **dadurch gekennzeichnet, dass** mindestens ein zusätzliches Abstützelement (12, 13) aus biegsamem oder elastischem Material auf jeder Federlamelle (8) montiert ist, vorzugsweise in einer entlang dieser Federlamelle (8) veränderbaren Position, wobei das oder die zusätzlichen Abstützelemente (12, 13) mit dem Rücken des Trägers oder des Benutzers in Berührung kommen.

10. Vorrichtung nach Anspruch 9, **dadurch gekennzeichnet, dass** ein oder zwei zusätzliche Abstützelemente (12, 13) aus biegsamem oder elastischem Material, zum Beispiel vom Typ aufblasbare Tasche mit viskoelastischem Körper, auf jeder Federlamelle (8) befestigt sind.

11. Vorrichtung nach Anspruch 10, **dadurch gekennzeichnet, dass** die Position der zusätzlichen Abstützelemente (12, 13) entlang der Federlamellen (8) einstellbar ist, also in der Höhe, durch eine Befestigung, deren Position durch ein selbsthaftendes System oder durch Einklicken auf den Rillen, die längs auf jeder Federlamelle (8) vorgesehen sind oder durch Gleiten entlang jeder Lamelle (8), zum Beispiel mit Hilfe eines Rings, einstellbar ist.

12. Vorrichtung nach einem der Ansprüche 9 bis 11, **dadurch gekennzeichnet, dass** die zusätzlichen Abstützelemente (12, 13) ausziehbar sind, vor allem durch Umklappen gegen die Rückseite jeder Federlamelle (8).

13. Vorrichtung nach einem der Ansprüche 9 bis 12, **dadurch gekennzeichnet, dass** die zusätzlichen Abstützelemente (12, 13) in unterschiedlichen Höhen auf jeder Federlamelle (8) befestigt sind, wobei jedes zusätzliche Abstützelement (12, 13) ein etwa dreieckiges Profil besitzt und vorzugsweise in umgekehrter Richtung zum anderen Element montiert ist.

14. Vorrichtung nach einem der Ansprüche 9 bis 13, **dadurch gekennzeichnet, dass** im Fall zusätzlicher Abstützelemente (12, 13) vom Typ aufblasbare Tasche diese in der Tiefe mit Hilfe einer für jede Tasche individuell aufblasbaren Birne (18, 19) einstellbar gestaltet sind.

15. Vorrichtung für Lordosestütze nach einem der Ansprüche 1 bis 14, **dadurch gekennzeichnet, dass** sie in einen Lumbalgürtel oder in ein orthopädisches Korsett eingearbeitet ist.

16. Vorrichtung für Lordosestütze nach einem der Ansprüche 1 bis 14, **dadurch gekennzeichnet, dass** sie mit einem oder mehreren speziellen Haltegurten (6) ausgestattet ist, die vorgesehen sind, den Rumpf ihres Trägers zu umfassen.

17. Vorrichtung für Lordosestütze nach einem der Ansprüche 1 bis 14, **dadurch gekennzeichnet, dass** sie vorgesehen ist, mit einem Sitz, insbesondere einer Rückenlehne eines Sitzes, zum Beispiel eines Kraftfahrzeugs, verbunden zu sein.
